(19) Europäisches Patentamt
European Patent Office
Office européen des brevets

(11)  EP 1 614 751 B1

(12)  **EUROPEAN PATENT SPECIFICATION**

(45) Date of publication and mention
of the grant of the patent:
**16.05.2007  Bulletin 2007/20**

(21) Application number: **04720630.5**

(22) Date of filing: **15.03.2004**

(51) Int Cl.:
*C12N 15/12* (2006.01)      *A61K 48/00* (2006.01)

(86) International application number:
**PCT/ES2004/000117**

(87) International publication number:
**WO 2004/090134 (21.10.2004 Gazette 2004/43)**

(54) **OLIGORIBONUCLEOTIDE SEQUENCE HOMOLOGOUS TO A CDNA REGION WHICH CODES FOR THE HUMAN CD40 RECEPTOR AND DUPLEX OLIGORIBONUCLEOTIDES, VECTORS, PHARMACEUTICAL COMPOSITIONS AND USES ASSOCIATED THERETO**

ZU EINEM FÜR DEN MENSCHLICHEN CD40-REZEPTOR CODIERENDEN CDNA-BEREICH HOMOLOGE OLIGORIBONUKLEOTIDSEQUENZ SOWIE DAMIT ZUSAMMENHÄNGENDE DUPLEX-OLIGORIBONUKLEOTIDE, VEKTOREN, PHARMAZEUTISCHE ZUSAMMENSETZUNGEN UND VERWENDUNGEN

SEQUENCE OLIGORIBONUCLEOTIDIQUE HOMOLOGUE A UNE REGION DE L'ADN C CODANT POUR LE RECEPTEUR HUMAIN CD40 ET OLIGORIBONUCLEOTIDES DUPLEX, VECTEURS, COMPOSITIONS PHARMACEUTIQUES ET UTILISATIONS CORRESPONDANTES

(84) Designated Contracting States:
**AT BE BG CH CY CZ DE DK EE ES FI FR GB GR HU IE IT LI LU MC NL PL PT RO SE SI SK TR**

(30) Priority: **08.04.2003  ES 200300822**

(43) Date of publication of application:
**11.01.2006  Bulletin 2006/02**

(73) Proprietors:
• **Aran Perramon, Josep Maria**
  **08100 Mollet del Vallés (ES)**
• **Grinyo Boira, Josep Maria**
  **08328 Alella (ES)**
• **Torras Ambros, Juan**
  **08018 Barcelona (ES)**
• **Pluvinet Ortega, Raquel**
  **08041 Barcelona (ES)**
• **Cruzado Garrit, Josep Maria**
  **08860 Castelldefels (ES)**
• **Herrero Fresneda, Immaculada**
  **08038 Barcelona (ES)**

(72) Inventors:
• **Aran Perramon, Josep Maria**
  **08100 Mollet del Vallés (ES)**

• **Grinyo Boira, Josep Maria**
  **08328 Alella (ES)**
• **Torras Ambros, Juan**
  **08018 Barcelona (ES)**
• **Pluvinet Ortega, Raquel**
  **08041 Barcelona (ES)**
• **Cruzado Garrit, Josep Maria**
  **08860 Castelldefels (ES)**
• **Herrero Fresneda, Immaculada**
  **08038 Barcelona (ES)**

(74) Representative: **Curell Aguilà, Marcelino et al**
  **Dr. Ing. M. Curell Sunol I.I. S.L.**
  **Passeig de Gràcia, 65 bis**
  **08008 Barcelona (ES)**

(56) References cited:
WO-A1-01/68860          WO-A1-03/006477
US-A1- 2002 132 257      US-B1- 6 197 584

• **RUSHWORTH, S.A. ET AL: 'Inhibition of CD40-mediated endothelial cell activation with antisense oligonucleotides'** TRANSPLANTATION vol. 73, no. 4, February 2002, pages 635 - 642, XP008040527

**Description**

Field of the Invention

The invention relates to an oligoribonucleotide sequence homologous to a region of cDNA coding for the human CD40 receptor, as per GeneBank accession number X60592, as well as to duplex oligoribonucleotides formed by the joining of said oligoribonucleotide sequences and sequences complementary thereto. A further object of the invention is also to provide pharmaceutical compositions comprising said oligoribonucleotide sequences and/or said duplex oligoribonucleotides, transfer vectors capable of expressing them, and uses thereof.

State of the Art

**Gene expression inhibition strategies**

At molecular level, diseases are frequently the result of the inadequate expression of one or various proteins, coded by the genes. The conventional pharmacological treatments applied up to date have, in many cases, managed to alleviate the symptomatology of the diseases, but the majority of said treatments involve side effects which are aggravated when the treatments are extended, due to the unspecific actions of the drugs employed.

With the development of molecular biology and genetic engineering there was recently introduced the concept of gene therapy, a specific causal treatment for disease based on direct actions on the nucleic acids. Thus, in cases where it is known that a particular viral, inflammatory or tumorous disease is due to an anomalous expression/synthesis of a particular protein, there has been developed a number of therapeutic methodologies called "antisense", directed to attenuating the production of said protein. To this end, small fragments of nucleic acids (antisense oligonucleotides), some of them having catalytic action (ribozymes, DNAzymes, ...) potentiating the degradation of the messenger RNA (mRNA) and/or blocking the biosynthesis of the protein involved in the disease by bonding specifically to certain sequences of the corresponding target mRNA are used. Nevertheless, in the preclinical and clinical tests carried out up to date, the inhibitory efficiency and the biological effects of said molecules have been questioned for there deficient ratio in the efficiency/safety balance.

Very recently, a promising new strategy for regulating or blocking the gene expression at RNA level, called "post-transcriptional gene silencing" (PTGS) has been introduced. Also called "RNA interference" (RNAi), said phenomenon relates to the specific suppression of the gene expression mediated by small double strand RNA molecules (siRNA). Very recent experimental results have proposed an RNAi actuation mechanism different from the one used by the antisense molecules. The siRNA molecules (preferably 21-25 nucleotides long) apparently act as a guide sequence which binds to a protein complex having nuclease activity. This complex is positioned in the target mRNA, which contains a specific sequence fully complementary to the antisense strand of the siRNA, and initiates degradation thereof.

The main advantage offered by the use of siRNAs optimized for RNAi relative to the "antisense" methods is their extreme efficacy, allowing very low concentrations of siRNA (from 1 to 100 nM), capable of producing an 80-100% specific inhibition of the expression of a particular gene, to be used. Furthermore, the siRNAs appear to be more resistant than the antisense oligonucleotides to the nuclease mediated degradation.

Therefore, the siRNAs may turn out to be very effective as therapeutic agents. The single strand oligoribonucleotide sequences are also known to be capable of producing a powerful specific inhibition of the expression of their target gene.

**The CD40 receptor as target for gene therapy treatments**

The protein CD40, identified in 1985, is a receptor located on the cell surface belonging to the family of tumorous necrosis factor receptors (TNF-R). Said biomolecule was initially characterized in the B cells. It was subsequently shown to play a key role in the T-cell-dependent humorous immune responses on analyzing the consequences of its dysfunction in patients having the hyper-IgM syndrome, and in *knock-out* mice (deficient) for CD40. Furthermore, its specific ligand, the protein CD40L (CD154), is a member of the family of tumorous necrosis factor (TNF) cytokines and is expressed fundamentally in CD4+-activated T cells. Thus, the CD40-CD40L interactions were observed to activate different signal transduction routes inducing the production of different cytokines and other mediators which, in a word, play a fundamental role in the regulation of the immune response.

It has recently been established that CD40 is also expressed in monocytes, dendritic cells, endothelial cells, fibroblasts and epithelial cells. In non-haematopoietic cells, the CD40 receptor is involved in the amplification and regulation of the inflammatory responses. Furthermore, experimental studies using *knock-out* mice or anti-CD40 antibodies have shown that the CD40-CD40L interaction blockade is beneficial in various pathological models, including transplants, autoimmune manifestations and infectious diseases.

Further information on the studies and techniques cited above may be obtained from the documents listed at the end of this description.

Summary of the Invention

An object of the invention is an oligoribonucleotide sequence of the group formed by the oligoribonucleotide sequences
5'-gcgaauuccuagacaccug-3' (SEQ ID N° 1)
and
5'-caggugucuaggaauucgc-3' (SEQ ID N° 2)
where these oligoribonucleotide sequences are homologous to the region comprised between the nucleotides 241 to 259 in the cDNA coding for the human CD40 receptor, as per GeneBank accession number X60592.
It is also an object of the invention to provide a duplex oligoribonucleotide formed by the joining by homology (also called complementary pairing) of the oligoribonucleotide sequences according to claim 1, where the resulting double strand is extended at the 3' ends by two unpaired terminal nucleotides uu or tt.

<div align="center">

**5'-gcgaauuccuagacaccuguu-3'**      **(SEQ ID N° 7)**

**3'-uucgcuuaaggaucuguggac-5'**


**5'-gcgaauuccuagacaccugtt-3'**      **(SEQ ID N° 8)**

**3'-ttcgcuuaaggaucuguggac-5'**

</div>

In the present application, the term "oligoribonucleotide sequence" is a term relating to the composition of an oligoribonucleotide. The oligoribonucleotides are biological elements or entities (short segments of RNA), being of necessity single strand. Likewise in this application, the term "duplex oligoribonucleotide" relates to the double strand oligoribonucleotides, such as for example siRNA, shRNA, etc.
Preferably the oligoribonucleotide sequence is extended at either of the ends thereof by at most four additional ribonucleotides in all; forming an extended oligoribonucleotide sequence, where said additional ribonucleotides are homologous to the corresponding region comprised between the nucleotides 237 a 263 in the cDNA coding for the human CD40 receptor, as per GeneBank accession number X60592, i.e.:
5'-gaaagcgaauuccuagacaccuggaac-3' (SEQ ID NO 13)
In fact, the addition of these additional ribonucleotides does not substantially modify the efficacy of the sequence. The invention also comprises the duplex oligoribonucleotides formed by the joining by homology of these extended oligoribonucleotide sequences, where the resulting double strand is extended at the 3' ends by two unpaired terminal nucleotides uu or tt. These terminal nucleotides uu or tt are obtained depending on the synthesis process employed: enzymatic (uu) or chemical (tt).
It is also possible to use oligoribonucleotide sequences which are formed on the one hand by the aforementioned oligoribonucleotide sequences, from which from 1 to 6 ribonucleotides have been removed from one of the ends, and the same number of oligoribonucleotides has been added at the other end. It is additionally possible to lengthen the resulting sequence with a number of oligoribonucleotides ranging from 1 to 6. In other words, it is also an object of the invention to provide an oligoribonucleotide sequence comprising an oligoribonucleotide subsequence of the group formed by the oligoribonucleotide subsequences
5'-uccuagacaccug-3' (SEQ ID N° 3)
5'-gcgaauuccuaga-3' (SEQ ID NO 4)
5'-ucuaggaauucgc-3' (SEQ ID N° 5)
5'-caggugucuagga-3' (SEQ ID NO 6)
and which additionally comprises a maximum of 12 additional ribonucleotides added at either of the ends thereof or at both ends simultaneously, forming a modified oligoribonucleotide sequence, such that the modified oligoribonucleotide sequence is homologous to the region comprised between the nucleotides 230 to 270 in the cDNA coding for the human CD40 receptor, as per GeneBank accession number X60592. It is also advantageous to provide a duplex oligoribonucleotide formed by the joining by homology of two modified oligoribonucleotide sequences such as those cited above, where the resulting double strand is extended at the 3' ends thereof by two unpaired terminal nucleotides uu or tt.
Advantageously, any of the oligoribonucleotide sequences according to the invention comprises additionally from one to three terminal ribonucleotides at the 3' end thereof, forming an elongated nucleotide sequence, where said terminal ribonucleotides are not homologous to the corresponding region between the nucleotides 230 to 270 in the cDNA coding for the human CD40 receptor, as per GeneBank accession number X60592. It is also advantageous to provide the corresponding duplex oligoribonucleotide formed by the joining by homology of two elongated oligoribonucleotide sequences, where the resulting double strand is extended at the 3' ends thereof by two unpaired terminal nucleotides uu or tt.

Preferably the oligoribonucleotide sequence, the extended oligoribonucleotide sequence, the modified oligoribonucleotide sequence, or the elongated oligoribonucleotide sequence has the joining of at least one peptide, one sugar, one lipid or one polymer. Also of interest are the duplex oligoribonucleotides formed from the same.

Advantageously the oligoribonucleotide sequence, the extended oligoribonucleotide sequence, the modified oligoribonucleotide sequence, or the elongated oligoribonucleotide sequence, or the corresponding duplex oligoribonucleotide have at least one chemical modification in one or another of the elements forming the structure thereof, either in the ribonucleosides or in the internucleotide bonds. This chemical modification is preferably a modification of the group formed by the addition of a phosphate group at the 5' end of the oligoribonucleotide sequence, the replacement by 2'-O-methyl groups of the 2'-hydroxyl groups in the component riboses of the oligoribonucleotide sequence, the substitution of ribonucloside analogs such as the N3' $\rightarrow$ P5' phosphoramidates for ribonucleosides, and the replacement of the internucleotide phosphate bonds by other bonds such as phosphorothioate or methylphosphonate. These modifications may be easily carried out and do not involve a substantial alteration of the biological properties.

A further preferred embodiment of the invention is obtained when in the duplex oligoribonucleotide of the invention the oligoribonucleotide sequences, the extended oligoribonucleotide sequences, the modified oligoribonucleotide sequences, or the elongated oligoribonucleotide sequences are covalently joined together at one of the ends thereof through a loop or linker of 2 to 25 nucleotides to form a shRNA.

It is also an object of the invention to provide a pharmaceutical composition comprising an oligoribonucleotide sequence and/or a duplex oligoribonucleotide according to the invention.

Yet a further object of the invention is to provide a gene transfer vector capable of inducing the expression of an oligoribonucleotide sequence and/or a duplex oligoribonucleotide according to the invention.

Advantageously there is used an oligoribonucleotide sequence and/or a duplex oligoribonucleotide according to the invention for the preparation of a medicament, said medicament being preferably for inhibiting the expression of the human CD40 receptor, or for blocking or reducing the human CD40-CD40L interaction.

It is likewise advantageous to use an oligoribonucleotide sequence and/or a duplex oligoribonucleotide according to the invention for the preparation of a medicament for the treatment of a disease of the group formed by reject phenomena in organ or tissue transplants, acute inflammatory processes, chronic inflammatory processes, processes with co-stimulation of lymphocytes, allergy manifestations, autoimmune manifestations or cardiovascular manifestations (such as, for example preferably, arteriosclerosis), infectious processes, rheumatologic manifestations and cancer.

A further preferred use of an oligoribonucleotide sequence and/or a duplex oligoribonucleotide according to the invention is for the preparation of a medicament for the treatment of a disease of the group formed by allergic dermatitis, psoriasis, arteriosclerosis, multiple sclerosis, pulmonary fibrosis, viral infections, bacterial infections, asthma, type I diabetes, Lyme disease, Crohn disease, ulcerous colitis, lupus, thyroiditis, arthritis, lymphomas and leukaemia.

It is also advantageous to use an oligoribonucleotide sequence and/or a duplex oligoribonucleotide according to the invention for the preparation of a medicament for the treatment of neurodegenerative disorders.

Brief Description of the Drawings

Further advantages and features of the invention will be disclosed in the following description in which, without any limiting nature, there is related a preferred embodiment of the invention, with reference to the accompanying drawings, in which:

Figure 1 is a graph of the distribution of four duplex siRNAs designed to inhibit the expression of human CD40 along the sequence of the corresponding cDNA.

Figure 2 shows CD40/cyclophyllin ratios obtained in the relative quantification of CD40 transcripts.

Figure 3 shows a Western blot analyses of the expression of CD40 (a) and β-actin (b) in the ECV-304 cells treated with the different siRNAs directed against human CD-40.

Figure 4 shows the expression of ICAM-1 in untreated HUVEC, by flow cytometry, unstimulated (light histogram) and stimulated with CD154 (dark histogram).

Figure 5 shows the expression of VCAM-1 in untreated HUVEC, by flow cytometry, unstimulated (light histogram) and stimulated with CD154 (dark histogram).

Figure 6 shows the expression of E-selectin in untreated HUVEC, by flow cytometry, unstimulated (light histogram) and stimulated with CD154 (dark histogram).

Figure 7 shows the expression of ICAM-1 in HUVEC stimulated with CD154 and transfected with siRNA-2 (light histogram) or with msiRNA-2 (dark histogram).

Figure 8 shows the expression of VCAM-1 in HUVEC stimulated with CD154 and transfected with siRNA-2 (light histogram) or with msiRNA-2 (dark histogram).

Figure 9 shows the expression of E-selectin in HUVEC stimulated with CD154 and transfected with siRNA-2 (light histogram) or with msiRNA-2 (dark histogram).

Figure 10 shows the percentage expression of ICAM-1 in stimulated HUVEC transfected with siRNA-2 or with msiRNA-2, relative to the non-transfected ones.

Figure 11 shows the percentage expression of VCAM-1 in stimulated HUVEC transfected with siRNA-2 or with msiRNA-2, relative to the non-transfected ones.

Figure 12 shows the percentage expression of E-selectin in stimulated HUVEC transfected with siRNA-2 or with msiRNA-2, relative to the non-transfected ones.

Figures 13 to 18 are phase-contrast microscopic images of various single layers of HUVEC, untreated HUVEC (Figure 13); untreated HUVEC stimulated with CD154 (Figure 14); untreated HUVEC incubated with HL-60 cells (Figure 15); untreated HUVEC stimulated with CD154 and incubated with HL-60 cells (Figure 16); HUVEC treated with msiRNA-2, stimulated with CD154 and incubated with HL-60 cells (Figure 17); HUVEC treated with siRNA-2, stimulated with CD154 and incubated with HL-60 cells (Figure 18).

Figure 19 shows the fluorescence ratio of adhered HL-60 cells and HUVEC cells transfected with siRNA-2 or msiRNA-2.

Experimental Results

The original results given below include the design, synthesis and characterization of the inhibitory efficacy of siRNAs for genetic silencing of CD40. There is shown the preparation of an siRNA (siRNA-2) having a potent inhibitory activity specific for the expression / synthesis of CD40 in a tumorous cell model expressing the CD40 receptor.

**Preparation of siRNAs against the human CD40 receptor.**

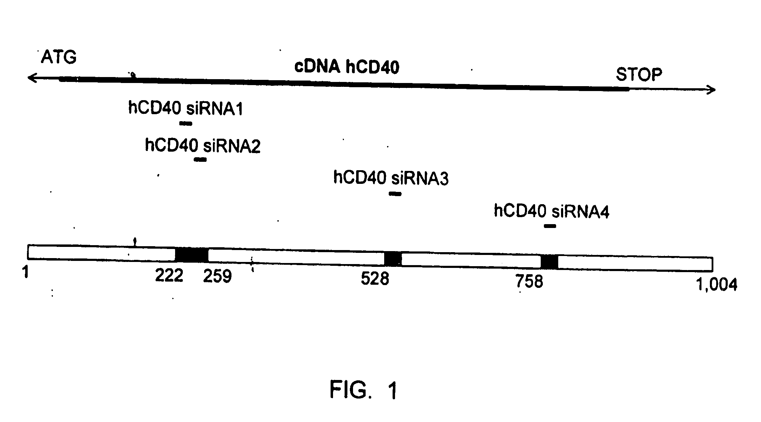

Duplex siRNAs directed against human CD40, plus a control duplex siRNA, the sequence of which is not directed to silence the expression of any gene, have been designed and synthesised. The methods used in screening the different synthesised siRNA molecules are described below. As an example, the complete characterization of four of said molecules is described in detail. The distribution of four of the duplex siRNAs designed to inhibit the expression of human CD40 along the sequence of the corresponding cDNA is shown in Figure 1.
To design the siRNAs from the sequence of the target cDNA, the non-translated 5' and 3' regions and the regions close to the start codon are avoided, since they have binding sites to regulating proteins. These proteins, as well as the initiation complexes of the protein translation may interfere with the binding of the RISC complex (RNA-induced silencing complex) having endonuclease activity, mediator in the degradation of the target mRNA.
For each siRNA a pair of oligonucleotides having 21 pairs of bases, 19 of which are complementary to the target gene, in this case human CD40, are used.
Fundamentally, there are two ways of preparing the siRNA duplexes *in vitro,* the chemical method, in which the strands of RNA of 21-25 nucleotides are separately synthesised by means of a conventional RNA/DNA synthesiser and are subsequently ringed by complementariness to form the siRNA duplex. Alternatively, there is used an enzyme method of siRNA synthesis based on the transcription *in vitro* using T7 RNA polymerase. In this case, the second method was used, *in vitro* transcription, since it is more economical for small scale characterization experiments.
Within the human CD40 cDNA sequence, there are sought sequences of the $AA(N_{19})$ type and once selected, there is added to the 3' end of each of the selected oligonucleotides a sequence of 8 pairs of bases complementary to the sequence of the T7 promoter (5'-cctgtctc-3'). These oligonucleotides and their complementary homologs are separately bonded to the primer of the T7 promoter, which in the presence of DNA polymerase (Klenow) generates a double strand DNA in each case. Subsequently, these short DNA fragments are used as substrates for an *in vitro* transcription reaction. Finally, the products of said transcription reactions are mutually hybridized generating double strand RNAs, also known as duplex siRNAs. The sequences of four synthesised duplex siRNAs, directed against human CD40, plus that of the control siRNA are as follows:

hCD40 siRNA-1

5'-uuucaccgcaaggaaggcauu-3'          (SEQ ID Nº 9)

3'-uuaaaguggcguuccuuccgu-5'

hCD40 siRNA-2

5'-caggugucuaggaauucgcuu-3'          (SEQ ID Nº 7)

3'-uuguccacagauccuuaagcg-5'

hCD40 siRNA-3

5'-agcuuguccaagggugacauu-3'          (SEQ ID Nº 10)

3'-uuucgaacagguucccacugu-5'

hCD40 siRNA-4

5'-aggaagaucgucgggaaaauu-3'          (SEQ ID Nº 11)

3'-uuuccuucuagcagcccuuuu-5'

Control siRNA

5'-uggucacgagucuuguaguuu-3'          (SEQ ID Nº 12)

3'-uuaccagugcucagaacauca-5'

**Efficacy of the duplex siRNAs in the silencing of the expression of human CD40 at mRNA level**

ECV-304 cells, a human cell line having endothelial features, were transfected (incubated) separately with the different duplex siRNAs directed against the mRNA for human CD40, including the control duplex siRNA, at a concentration of 100nM in each case. Penetration thereof inside the cells was facilitated by using a cationic lipid as transfer vehicle. After 48 hours, the levels of expression of the CD40 transcript in all the transfected specimens were analyzed by real time RT-PCR. These values were normalized relative to the expression of a reference gene (the cyclophyllin gene was taken here as reference). Thus, the CD40/cyclophyllin expression ratio was calculated.

In this semi-quantitative analysis, the expression of the target gene (CD40) of each of the specimens treated with siRNAs was expressed as a value relative to the specimen treated with the control siRNA. The quantification result is given in relative units of expression.

Table 1 below shows the CD40 / cyclophyllin ratios obtained in the relative quantification of CD40 transcripts.

Table 1

| CD40/cyclophyllin ratio (mean ± standard deviation) | |
| --- | --- |
| | ECV-304 |
| | 100nM |
| **hCD40-siRNA-1** | 3.41 ± 1.09 |
| **hCD40-siRNA-2** | 0.71 ± 0.61* |
| **hCD40-siRNA-3** | 3.38 ± 0.69 |
| **hCD40-siRNA-4** | 4.55 ± 2.09 |
| **Control siRNA** | 2.82 ± 1.07 |

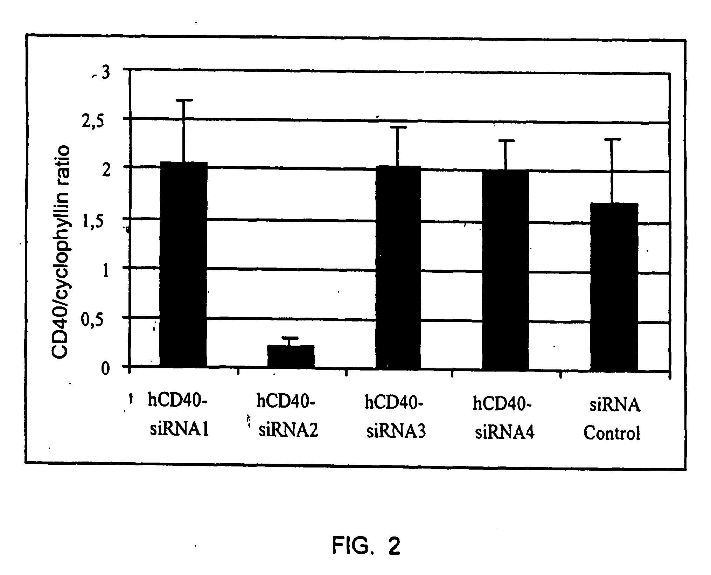

Thus, according to the results obtained, the duplex siRNA-2 of the present invention causes a significant inhibition ($p < 0.05$; Student "T test") of about four times of the expression of CD40 over the value obtained in the cells treated with the control siRNA. Figure 2 shows these results graphically.

**Efficacy of the duplex siRNAs in the inhibition of the synthesis of the human CD40 receptor.**

In this case, the ECV-304 cells were also transfected with the siRNAs (100 nM) directed against human CD40 (and in parallel with the corresponding control siRNA). They were then stimulated with the proinflammatory cytokines TNF-$\alpha$ and IF-$\gamma$, which induce the overexpression of CD-40. 24 hours after treatment, the cells were lysed and the expression levels of CD40 (50 kDa) were analyzed in the protein extract by Western blot, with a specific antibody against human CD40. The obtained values were normalized relative to the expression of $\beta$-actin (42 kDa) in said cells.

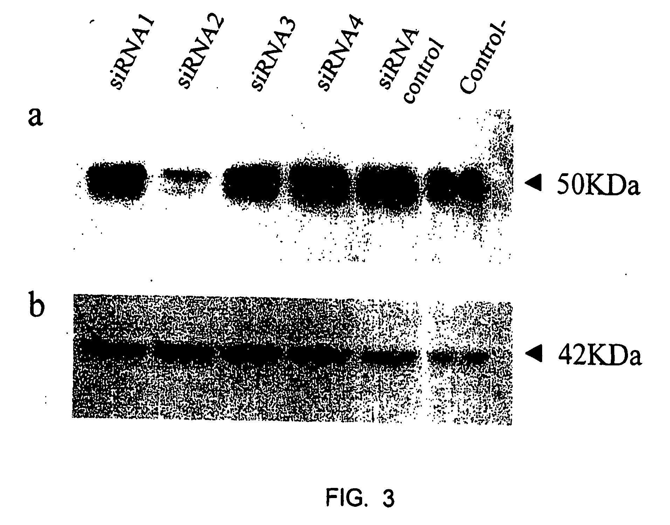

The results obtained are given in Figure 3. It is confirmed that only in the cells treated with the duplex siRNA-2, and not with the cells treated with any of the other siRNAs analyzed, was any great silencing of the expression of CD40 induced. In the case of the siRNA-2 of the present invention, the CD40 protein levels show a reduction of over 80% with respect to the untreated specimens, or treated with the control duplex siRNA.

**Inhibition by RNA interference (RNAi) of the expression of cellular adhesion molecules (CAM) in CD-154-activated endothelial cells. RNAi mediated inhibition of leukocyte adhesion in CD154-activated endothelial cells.**

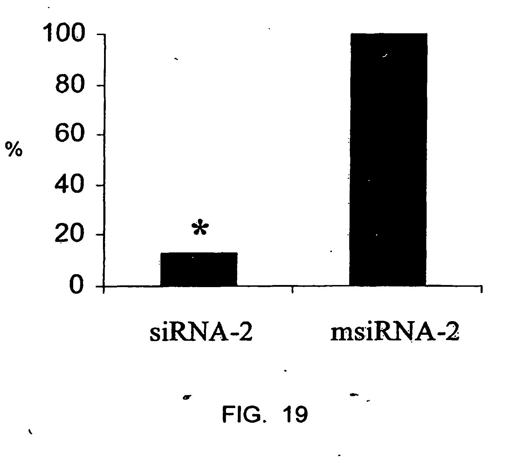

An important step in the immunoinflammatory process, the activation of the endothelial cells by the CD40-CD154 interaction, induces the expression of cellular adhesion molecules CAM which causes the exit of inflammatory cells from the lumen of the blood vessel. It was therefore investigated whether the siRNA-2 can interfere in the CD40-CD154 activation route in HUVEC (human umbilical vein endothelial cells). The effect of siRNA-2 versus a control, msiRNA-2, which is a siRNA sequence equal to that of siRNA-2, but with a subsequence of 5 pairs of bases altered at an intermediate point, was compared. The comparison was effected by flow cytometry analysis of both the cellular adhesion molecules CAM and the adherence of leukocytes in CD40-activated endothelial cells. When HUVEC were co-cultured with Jurkat D1.1 CD154+ cells, HUVEC increased the expressions of ICAM-1, VCAM-1 and E-selectin. On the contrary, HUVEC treated with siRNA-2 exhibited a powerful reduction in the expression of the above CAM when they were activated via CD154. The treatment of HUVEC with the control msiRNA-2 only brought about a slight reduction of the expression of the expression of CAM after activation by CD154. Furthermore, the direct evaluation of the adherence of leukocytes in CD154-activated HUVEC using HL-60 cells, which express the ligands for the three adhesion molecules ICAM-1, VCAM-1 and E-selectin showed that the pretreatment with siRNA-2 reduced the adherence of leukocytes by 87% (Figure 19), which is not achieved with the msiRNA.2. Therefore, the potent inhibitory effect of the siRNA-2 over the CD40-CD154 pathway is comparable with what was observed on employing an antibody blocking the CD154. Additionally, the anti-CD40 siRNA-2 has no effect on the adherence of leukocytes induced by activation of HUVEC by TNF-$\alpha$, demonstrating its incapability for interfering with CD-120 (another member of the TNF-$\alpha$ receptors) and, therefore, its specificity.

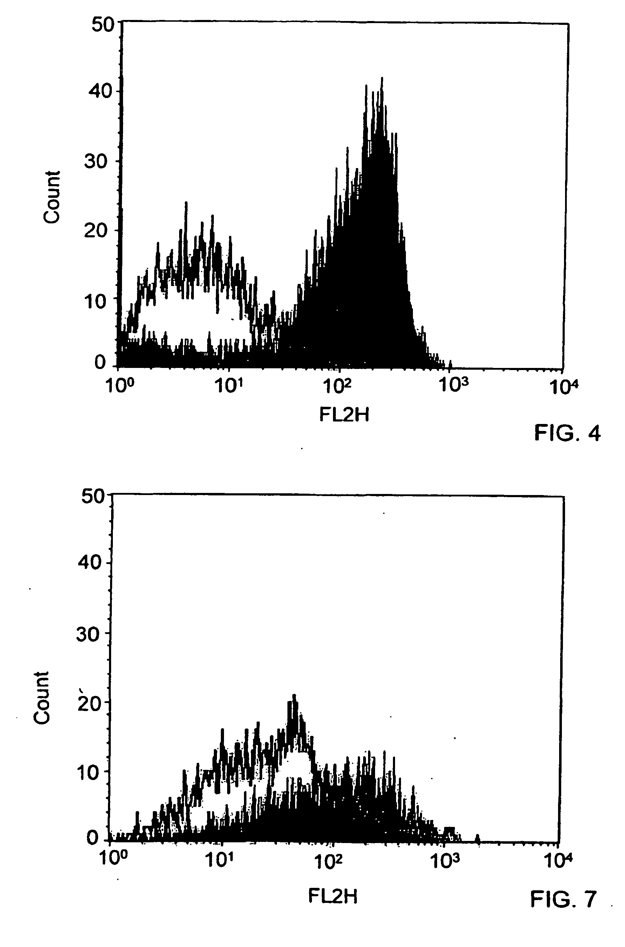

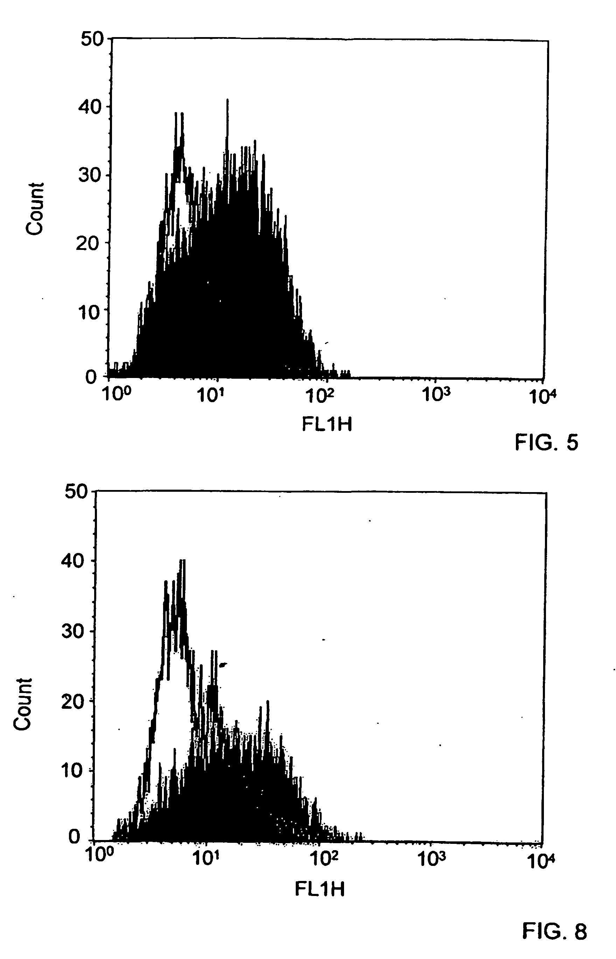

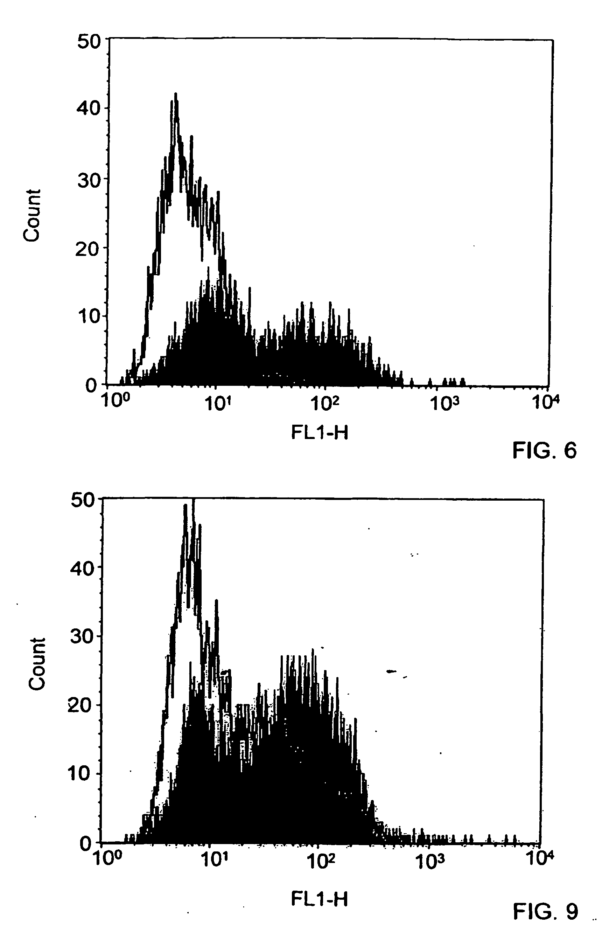

In a series of assays, HUVEC cells, both non-transfected and transfected with siRNA-2 or msiRNA-2, were activated by co-incubation with Jurkat D1.1 (CD154+) cells with a T.EC ratio of 5:1 for 6 hours (E-selectin) or 16 hours (ICAM-1 andVCAM-1) before the analysis. About 48 hours after transfection, the co-cultures were washed with PBS to remove the Jurkat D1.1 cells adhered to the single layer of HUVEC. The expression of E-selectin, ICAM-1 and VCAM-1 in the endothelial cells was analyzed by flow cytometry. In Figures 4, 5 and 6, the light histograms represent the basal expression of CAM for untreated HUVEC and the dark histograms represent the expression of CAM in untreated HUVEC stimulated with D1.1 (CD154+). In Figures 7, 8 and 9, the light histograms represent the expression of CAM in HUVEC transfected with siRNA-2, stimulated with D1.1 (CD154+), and the dark histograms represent the expression of CAM in HUVEC transfected with msiRNA-2, stimulated with D1.1 (CD154+).

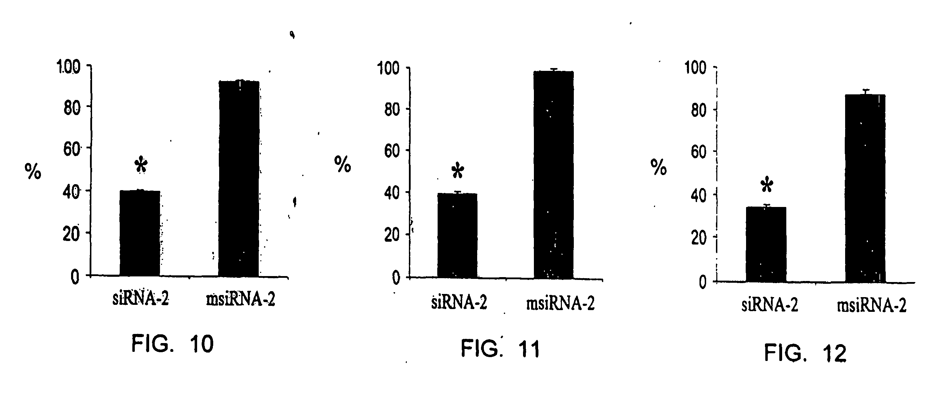

Figures 10, 11 and 12 show bar diagrams indicating the percentage expression of CAM calculated as follows:

$$[((\text{expression of CAM for cells treated with siRNA-2 and induced with D1.1 (CD154+)}) - (\text{expression of basal CAM})) / ((\text{expression of CAM for untreated cells induced with D1.1 (CD154+)}) - (\text{expression of basal CAM}))] \times 100.$$

Similar calculations have been made for msiRNA-2.

The CAM expression data are the mean fluorescence intensity (MFI) values of the corresponding histograms, for three independent experiments.

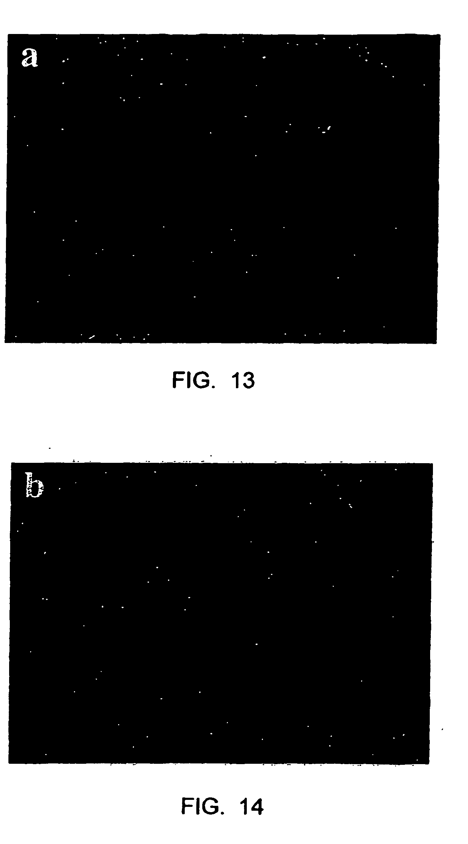

In a further series of assays, HUVEC cells, non-transfected or transfected with siRNA-2 or msiRNA-2, were activated by co-culturing with Jurkat D1.1 (CD154+) cells with a T.EC ratio of 5:1 for 16 hours before the assay. 48 hours after transfection, the co-cultures were washed with abundant PBS to remove the Jurkat D1.1 cells adhered to the single layer of HUVEC, which was subsequently co-incubated with calcein-AM-loaded HL-60 cells for 30 minutes at 37°C. The supernatant was removed by suction and two gentle washings were done with PBS to remove the non-adhered cells. Figures 13 to 18 show phase-contrast microscopic images with a 40X amplification, as follows:

Figure 13, single layer of untreated HUVEC.
Figure 14, single layer of untreated HUVEC, co-incubated with HL-60 cells.

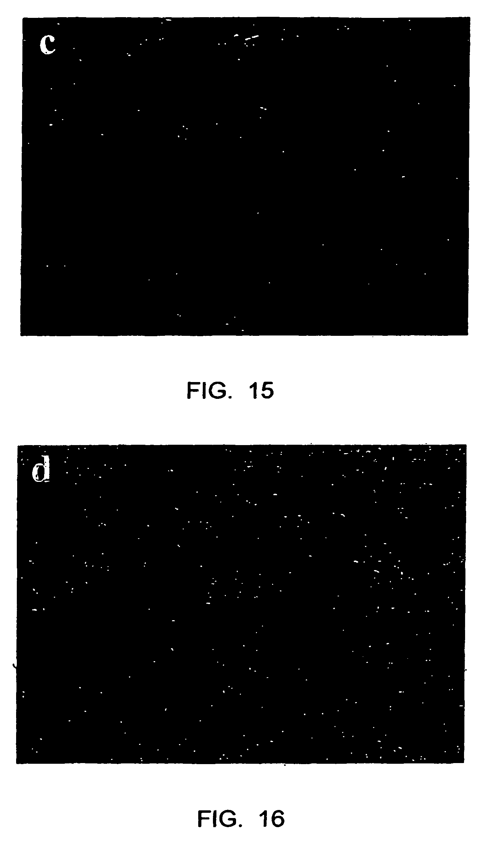

Figure 15, untreated HUVEC, activated with Jurkat D1.1 (CD154+).
Figure 16, untreated HUVEC, activated with Jurkat D1.1 (CD154+), co-incubated with HL-60 cells.

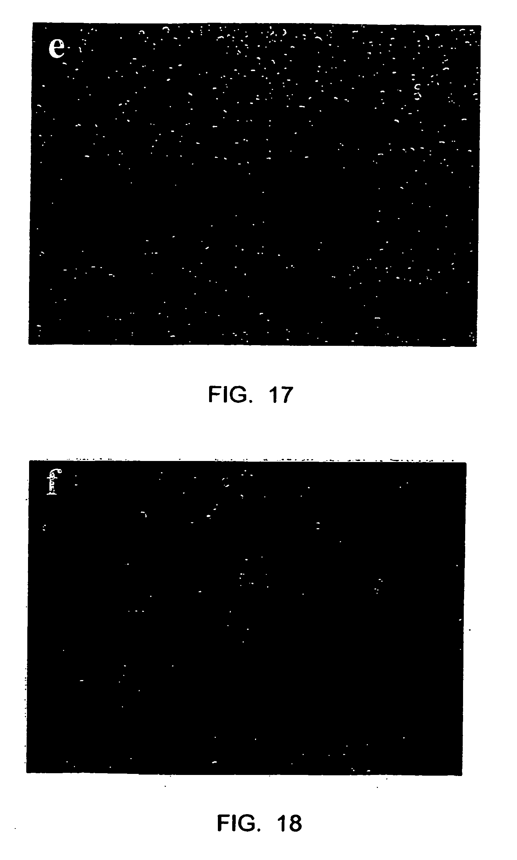

Figure 17, HUVEC treated with msiRNA-2, activated with Jurkat D1.1 (CD154+), co-incubated with HL-60 cells.
Figure 18, HUVEC treated with siRNA-2, activated with Jurkat D1.1 (CD154+), co-incubated with HL-60 cells.

After microscopic examination, the remaining adhered cells were collected and the ratio of fluorescent marked adhered HL-60 cells to non marked endothelial cells was determined. The ratio was determined by flow cytometry after counting 10,000 events per specimen. 100% adherence was assigned to the mean ratio obtained from HUVEC treated with msiRNA-2 (Figure 19).

**Indications**

The CD40-CD40L interaction plays a very important role in different aspects of the humoral and cellular immune response. Said interaction is essential in the activation opf the T cells, to enhance the capacity of presentation of antigen in the antigen presenting cells (APC), in the activation of the B cells (inducing their proliferation and in the control of the change of isotope of the IgGs), and in the activation of epithelial and endothelial cells involved in inflammatory processes.

Therefore, one use in accordance with the invention is the use for the preparation of medicaments having an anti human CD40 inhibitor in accordance with the invention, such as the siRNA-2, for the blockage or reduction of the CD40-CD40L interaction which is the basis of human pathology or is present therein in any degree, in any medical discipline and with the purpose of producing beneficial or therapeutical effects.

Therefore, the use of anti-CD40 siRNAs will be indicated especially in the field of organ transplants to inhibit or completely avoid the acute rejection phenomena and for the induction of long-term tolerance with the concomitant administration of other immunosuppressors or alone and isolated.

Furthermore, the blockage of the CD40-CD40L interaction by siRNAs may be adequate in therapeutical applications in any disease proceeding with inflammatory processes, such as allergy, autoimmune, cardiovascular, infectious and rheumatologic manifestations (allergic dermatitis, psoriasis, arteriosclerosis, multiple sclerosis, pulmonary fibrosis, viral and bacterial infections, asthma, type I diabetes, Lyme disease, Crohn disease, thyroiditis, arthritis), and even in neurodegenerative disorders such as Alzheimer's disease. Different preclinical studies that have employed anti-CD40L blocking antibodies, or *knock-out* mice both for CD40 and for CD40L, support these statements.

**General References**

- McManus, M.T., and Sharp, P.A. (2002) Gene silencing in mammals by small interfering RNAs. *Nature Rev.* **3**: 737-747.
- Elbashir, S.M., Harborth, J., Weber, K., and Tuschl, T. (2002) Analysis of gene function in somatic mammalian cells using small interfering RNAs. *Methods* **26**: 199-213.
- Bertrand, J.-R., Pottier, M., Vekris, A., Opolon, P., Maksimenko, A., and Malvy, C. (2002) Comparison of antisense oligonucleotides and siRNAs in cell culture and in vivo. *Biochem. Biophys. Res. Com.* **296**: 1000-1004.
- Xia, H., Mao, Q., Paulson, H.L., and Davidson, B.L. (2002) siRNA-mediated gene silencing *in vitro* and *in vivo*. *Nature Biotechnol.* **20**: 1006-1010.
- Elbashir, S.M., Lendeckel, W., and Tuschl, T. (2001) RNA interference is mediated by 21- and 22-nucleotide RNAs. *Genes & Dev.* **15**: 188-200.
- Hammond, S.M., Caudy, A.A., and Hannon, G.J. (2001) Post-transcriptional gene silencing by double-stranded RNA. *Nature Rev.* **2**: 110-119.
- Paddison, P.J., Caudy, A.A., Bernstein, E., Hannon, G.J., and Conklin, D.S. (2002) Short hairpin RNAs (shRNAs) induce sequence-specific silencing in mammalian cells. *Genes & Dev.* **16**: 948-958.
- Brummelkamp, T.R., Bernards, R., and Agami, R. (2002) A system for stable expression of short interfering RNAs in mammalian cells. *Science* **296**: 550-553.
- Paddison, P.J., Caudy, A.A., and Hannon, G.J. (2002) Stable suppression of gene expression by RNAi in mammalian cells. *Proc. Natl. Acad. Sci. USA* **99**: 1443-1448.
- Van Kooten, C., and Banchereau, J. (2000) CD40-CD40 ligand. J. *Leukocyte Biol.* **67**: 2-17.
- Denton, M.D., Reul, R.M., Dharnidharka, V.R., Fang, J.C., Ganz, P., and Briscoe, D.M. (1998) Central role for CD40/CD40 ligand (CD154) interactions in transplant rejection. *Pediatr. Transplantation* **2**: 6-15.
- Chess, L. (2001) "Blockade of the CD40UCD40 pathway", in *Therapeutic Immunology 2nd edition*. Austen, K.F., Burakoff, S., Rosen, F., and Strom, T., eds. Blackwell Science, Malden, MA, USA. pp.-441-456.
- Martinez, J., Patkaniowska, A., Urlaub, H., Lührmann, R., Tuschl, T., (2002) Single-stranded antisense siRNAs guide target RNA cleavage in RNAi. *Cell* **110**: 563-574

LIST OF SEQUENCES

<110 ARAN, JOSEP Mª
GRINYO, JOSEP Mª
TORRAS, JOAN
PLUVINET, RAQUEL
HERRERO, IMMACULADA
CRUZADO, JOSEP Mª

<120> Oligoribonucleotide sequence homologous to a region of the cDNA coding for the human CD40 receptor and duplex oligoribonucleotides, vectors, pharmaceutical compositions and corresponding uses.

<130> ARAN-GRINYO-TORRAS-Anti-CD40

<160> 13

<170> Patentin version 3.1

<210> 1
<211> 19
<212> RNA
<213> Homo sapiens

<220>
<221> mist_RNA
<222> (1)..(19)

<223> siRNA homologous to a region of the B cell CDS activation molecules; gene CD40; gene CDw40; nervous growth factor receptor, (241).... (259)

<400> 1
gcgaauuccu agacaccug          19

<210> 2
<211> 19
<212> RNA
<213> Homo sapiens

<220>
<221 > misc_RNA
<222> (1)..(19)
<223> siRNA complementary to a region of the B cell CDS activation molecules; gene CD40; gene CDw40; nervous growth factor receptor; (259) .... (241)

<400> 2
caggugucua ggaauucgc          19

<210> 3
<211> 13
<212> RNA
<213> Homo sapiens

<220>
<221> misc_RNA
<222> (1)..(13)
<223> siRNA homologous to a region of the B cell CDS activation molecules; gene CD40; gene CDw40; nervous growth factor receptor; (247).... (259)

<400> 3
uccuagacac cug          13

<210> 4
<211> 13
<212> RNA
<213> Homo sapiens

<220>
<221> misc_RNA
<222> (1)..(13)
<223> siRNA homologous to a region of the B cell CDS activation molecules; gene CD40; gene CDw40; nervous growth factor receptor; (241) .... (253)

<400> 4
gcgaauuccu aga          13

<210> 5
<211> 13
<212> RNA
<213> Homo sapiens

<220>
<221> misc_RNA
<222> (1)..(13)
<223> siRNA complementary to a region of the B cell CDS activation molecules; gene CD40; gene CDw40; nervous growth factor receptor, (253).... (241)

<400> 5
ucuaggaauu cgc          13


<210> 6
<211> 13
<212> RNA
<213> Homo sapiens


<220>
<221> misc_RNA
<222> (1)..(13)
<223> siRNA complementary to a region of the B cell CDS activation molecules; gene CD40; gene CDw40; nervous growth factor receptor; (259) ... (247).


<400> 6
caggugucua gga          13


<210> 7
<211> 21
<212> RNA
<213> Homo sapiens


<220>
<221 > misc_RNA
<222> (1)..(21)
<223> siRNA homologous to a region of the B cell CDS activation molecules; gene CD40; gene CDw40; nervous growth factor receptor; (241) .... (259)


<400> 7
gcgaauuccu agacaccugu u          21


<210> 8
<211> 21
<212> DNA/RNA<213> Homo sapiens


<220>
<221 > misc_RNA
<222> (1)..(21)
<223> siRNA homologous to a region of the B cell CDS activation molecules; gene CD40; gene CDw40; nervous growth factor receptor; (241) ..... (259)


<400> 8
gcgaauuccu agacaccugt t          21


<210> 9
<211> 21
<212> RNA
<213> Homo sapiens


<220>
<221 > misc_RNA
<222> (1)..(21)
<223> siRNA complementary to a region of the B cell CDS activation molecules; gene CD40; gene CDw40; nervous growth factor receptor; (240) ... (222)


<400> 9
uuucaccgca aggaaggcau u          21

<210> 10
<211> 21
<212> RNA
<213> Homo sapiens

<220>
<221> misc_RNA
<222> (1)..(21)
<223> siRNA complementary to a region of the B cell CDS activation molecules; gene CD40; gene CDw40; nervous growth factor receptor; (546) ... (528)

<400> 10
agcuugucca agggugacau u        21

<210> 11
<211> 21
<212> RNA
<213> Homo sapiens

<220>
<221 > misc_RNA
<222> (1)..(21)
<223> siRNA complementary to a region of the B cell CDS activation molecules; gene CD40; gene CDw40 ; nervous growth factor receptor ; (776) ... (758)

<400> 11
aggaagaucg ucgggaaaau u        21

<210> 12
<211> 21
<212> RNA
<213> Homo sapiens

<220>
<221> misc_RNA
<222> (1)..(21)
<223>

<400> 12
uggucacgag ucuuguaguu u        21

<210> 13
<211> 27
<212> RNA
<213> Homo sapiens

<220>
<221> misc_RNA
<222> (1)..(27)
<223> siRNA homologous to a region of the B cell CDS activation molecules; gene CD40; gene CDw40; nervous growth factor receptor; (237) ... (263)

<400> 13
gaaagcgaau uccuagacac cuggaac        27

**Claims**

1.  An oligoribonucleotide sequence of the group formed by the oligoribonucleotide sequences
    5'-gcgaauuccuagacaccug-3' (SEQ ID N° 1)
    and
    5'-caggugucuaggaauucgc-3' (SEQ ID N° 2)
    where these oligoribonucleotide sequences are homologous to the region comprised between the nucleotides 241 to 259 in the cDNA coding for the human CD40 receptor, as per GeneBank accession number X60592.

2.  An oligoribonucleotide sequence according to claim 1, **characterized in that** it is extended at either of the ends thereof by at most four additional ribonucleotides in all; forming an extended oligoribonucleotide sequence, where said additional ribonucleotides are homologous to the corresponding region comprised between the nucleotides 237 a 263 in the cDNA coding for the human CD40 receptor, as per GeneBank accession number X60592.

3.  An oligoribonucleotide sequence comprising an oligoribonucleotide subsequence of the group formed by the oligoribonucleotide subsequences
    5'-uccuagacaccug-3' (SEQ ID N° 3)
    5'-gcgaauuccuaga-3' (SEQ ID N° 4)
    5'-ucuaggaauucgc-3' (SEQ ID N° 5)
    5'-caggugucuagga-3' (SEQ ID N° 6)
    and which additionally comprises a maximum of 12 additional ribonucleotides added at either of the ends thereof or at both ends simultaneously, forming a modified oligoribonucleotide sequence, such that said modified oligoribonucleotide sequence is homologous to the region comprised between the nucleotides 230 to 270 in the cDNA coding for the human CD40 receptor, as per GeneBank accession number X60592.

4.  An oligoribonucleotide sequence according to any one of claims 1 to 3, **characterized in that** it comprises additionally from one to three terminal ribonucleotides at the 3' end thereof, forming an elongated nucleotide sequence, where said terminal ribonucleotides are not homologous to the corresponding region between the nucleotides 230 to 270 in the cDNA coding for the human CD40 receptor, as per GeneBank accession number X60592.

5.  An oligoribonucleotide sequence according to any one of claims 1 to 4, **characterized in that** said oligoribonucleotide sequence, said extended oligoribonucleotide sequence, said modified oligoribonucleotide sequence, or said elongated oligoribonucleotide sequence has the joining of at least one peptide, one sugar, one lipid or one polymer.

6.  An oligoribonucleotide sequence according to any one of claims 1 to 5, **characterized in that** said oligoribonucleotide sequence, said extended oligoribonucleotide sequence, said modified oligoribonucleotide sequence, or said elongated oligoribonucleotide sequence has one chemical modification in one or another of the elements forming the structure thereof, either in the ribonucleosides or in the internucleotide bonds, where said chemical modification is a modification of the group formed by the addition of a phosphate group at the 5' end of the oligoribonucleotide sequence, the replacement by 2'-O-methyl groups of the 2'-hydroxyl groups in the component riboses of the oligoribonucleotide sequence, the substitution of ribonucloside analogs such as the N3'→P5' phosphorimadates, for ribonucleosides and the replacement of the internucleotide phosphate bonds by other bonds such as phosphorothioate or methylphosphonate.

7.  A duplex oligoribonucleotide formed by the joining by homology of the oligoribonucleotide sequences according to claim 1, where the resulting double strand is extended at the 3' ends thereof by two unpaired terminal nucleotides uu or tt.

$$\text{5'-gcgaauuccuagacaccuguu-3'} \qquad \text{(SEQ ID N° 7)}$$
$$\text{3'-uucgcuuaaggaucuguggac-5'}$$

$$\text{5'-gcgaauuccuagacaccugtt-3'} \qquad \text{(SEQ ID N° 8)}$$
$$\text{3'-ttcgcuuaaggaucuguggac-5'}$$

8. A duplex oligoribonucleotide formed by the Joining by homology of the extended oligoribonucleotide sequences according to claim 2, where the resulting double strand is extended at the 3' ends thereof by two unpaired terminal nucleotides uu or tt.

9. A duplex oligoribonucleotide formed by the joining by homology of two modified oligoribonucleotide sequences according to claim 3, where the resulting double strand is extended at the 3' ends thereof by two unpaired terminal nucleotides uu or tt.

10. A duplex oligoribonucleotide formed by the joining by homology of two elongate oligoribonucleotide sequences according to claim 4, where the resulting double strand is extended at the 3' ends thereof by two unpaired terminal nucleotides uu or tt.

11. A duplex oligoribonucleotide according to any one of claims 7 to 10,
**characterized in that** said oligoribonucleotide sequence, said extended oligoribonucleotide sequence, said modified oligoribonucleotide sequence, or said elongated oligoribonucleotide sequence has the joining of at least one peptide, one sugar, one lipid or one polymer.

12. A duplex oligoribonucleotide according to any one of claims 7 to 11, **characterized in that** said oligoribonucleotide sequence, said extended oligoribonucleotide sequence, said modified oligoribonucleotide sequence, or said elongated oligoribonucleotide sequence has at least one chemical modification in one or another of the elements forming the structure thereof, either in the ribonucleosides or in the internucleotide bonds, where said chemical modification is a modification of the group formed by the addition of a phosphate group at the 5' end of the oligoribonucleotide sequence, the replacement by 2'-O-methyl groups for 2'-hydroxy groups in the component riboses of the oligoribonucleotide sequence, the substitution of ribonucloside analogs, such as the N3'$\rightarrow$P5' phosphorimadates, for ribonucleosides and the replacement of the internucleotide phosphate bonds by other bonds such as phosphorothioate or methylphosphonate.

13. A duplex oligoribonucleotide according to any one of claims 7 to 12, **characterized in that** said oligoribonucleotide sequences, said extended oligoribonucleotide sequences, said modified oligoribonucleotide sequences, or said elongated oligoribonucleotide sequences are covalently joined together at one of the ends thereof through a loop or linker of 2 to 25 nucleotides to form an shRNA.

14. A pharmaceutical composition comprising an oligoribonucleotide sequence according to any one of claims 1 to 6.

15. A pharmaceutical composition comprising a duplex oligoribonucleotide according to any one of claims 7 to 13.

16. A gene transfer vector expressing an oligoribonucleotide sequence according to any one of claims 1 to 6.

17. A gene transfer vector expressing a duplex oligoribonucleotide according to any one of claims 7 to 13.

18. The use of an oligoribonucleotide sequence according to any one of claims 1 to 6 for the preparation of a medicament.

19. The use of a duplex oligoribonucleotide according to any one of claims 7 to 13 for the preparation of a medicament.

20. The use of an oligoribonucleotide sequence according to any one of claims 1 to 6 for the preparation of a medicament for the treatment of a disease of the group formed by reject phenomena in organ or tissue transplants, acute inflammatory processes, chronic inflammatory processes, processes with co-stimulation of lymphocytes, allergy manifestations, autoimmune manifestations or cardiovascular manifestations, infectious processes, rheumatologic manifestations and cancer.

21. The use of a duplex oligoribonucleotide according to any one of claims 7 to 13 for the preparation of a medicament for the treatment of a disease of the group formed by reject phenomena in organ or tissue transplants, acute inflammatory processes, chronic inflammatory processes, processes with co-stimulation of lymphocytes, allergy manifestations, autoimmune manifestations or cardiovascular manifestations, infectious processes, rheumatologic manifestations and cancer.

22. The use of an oligoribonucleotide sequence according to any one of claims 1 to 6 for the preparation of a medicament for the treatment of a disease of the group formed by allergic dermatitis, psoriasis, arteriosclerosis, multiple sclerosis, pulmonary fibrosis, viral infections, bacterial infections, asthma, type I diabetes, Lyme disease, Crohn disease, ulcerous colitis, lupus, thyroiditis, arthritis, leukemias and lymphomas.

23. The use of a duplex oligoribonucleotide according to any one of claims 7 to 13 for the preparation of a medicament for the treatment of a disease of the group formed by allergic dermatitis, psoriasis, arteriosclerosis, multiple sclerosis, pulmonary fibrosis, viral infections, bacterial infections, asthma, type I diabetes, Lyme disease, Crohn disease, ulcerous colitis, lupus, thyroiditis, arthritis, leukemias and lymphomas.

24. The use of an oligoribonucleotide sequence according to any one of claims 1 to 6 for the preparation of a medicament for the treatment of neurodegnerative disorders.

25. The use of a duplex oligoribonucleotide according to any one of claims 7 to 13 for the preparation of a medicament for the treatment of neurodegnerative disorders.

**Patentansprüche**

1. Oligoribonukleotidsequenz aus der Gruppe gebildet durch die Oligoribonukleotidsequenzen
   5'-gcgaauuccuagacaccug-3' (SEQ. ID. NR. 1)
   und
   5'-cagguguguaggaauucgc-3' (SEQ. ID. NR. 2)
   wobei diese Oligoribonukleotidsequenzen homolog zu der Region eingeschlossen zwischen den Nukleotiden 241 bis 259 in der cDNA sind, welche den humanen CD40-Rezeptor, wie laut GeneBank-Zugangsnummer X60592, codiert.

2. Oligoribonukleotidsequenz nach Anspruch 1, **dadurch gekennzeichnet, dass** sie an einem der Enden davon durch insgesamt höchstens vier zusätzliche Ribonukleotide erweitert ist, wodurch eine erweiterte oligoribonukleotidsequenz gebildet wird, wobei die zusätzlichen Ribonukleotide homolog zu der entsprechenden Region eingeschlossen zwischen den Nukleotiden 237 bis 263 in der cDNA sind, welche den humanen CD40-Rezeptor, wie laut GeneBank-Zugangsnummer X60592, codiert.

3. Oligoribonukleotidsequenz, welche eine Oligoribonukleotidsubsequenz aus der Gruppe gebildet durch die Oligoribonukleotidsubsequenzen
   5'-uccuagacaccug-3' (SEQ. ID. NR. 3)
   5'-gcgaauuccuaga-3' (SEQ. ID. NR. 4)
   5'-ucuaggaauucgc-3' (SEQ. ID. NR. 5)
   5'-cagguguguagga-3' (SEQ. ID. NR. 6)
   umfasst, und welche außerdem ein Maximum von 12 zusätzlichen Ribonukleotiden hinzugefügt an einem der Enden davon oder an beiden Enden gleichzeitig umfasst, wodurch eine modifizierte Oligoribonukleotidsequenz gebildet wird, so dass die modifizierte Oligoribonukleotidsequenz homolog zu der Region eingeschlossen zwischen den Nukleotiden 230 bis 270 in der cDNA ist, welche den humanen CD40-Rezeptor, wie laut GeneBank-Zugangsnummer X60592, codiert.

4. Oligoribonukleotidsequenz nach einem der Ansprüche 1 bis 3, **dadurch gekennzeichnet, dass** sie zusätzlich ein bis drei Abschlussribonukleotide am 3'-Ende davon umfasst, wodurch eine verlängerte Nukleotidsequenz gebildet wird, wobei die Abschlussribonukleotide nicht homolog zu der entsprechenden Region zwischen den Nukleotiden 230 bis 270 in der cDNA sind, welche den humanen CD40-Rezeptor, wie laut GeneBank-Zugangsnummer X60592, codiert.

5. Oligoribonukleotidsequenz nach einem der Ansprüche 1 bis 4, **dadurch gekennzeichnet, dass** die Oligoribonukleotidsequenz, die erweiterte Oligoribonukleotidsequenz, die modifizierte Oligoribonukleotidsequenz oder die verlängerte Oligoribonukleotidsequenz die Verbindung von mindestens einem Peptid, einem Zucker, einem Lipid oder einem Polymer aufweist.

6. Oligoribonukleotidsequenz nach einem der Ansprüche 1 bis 5, **dadurch gekennzeichnet, dass** die Oligoribonukleotidsequenz, die erweiterte Oligoribonukleotidsequenz, die modifizierte Oligoribonukleotidsequenz oder die verlängerte Oligoribonukleotidsequenz eine chemische Modifikation in einem oder einem anderen der Elemente aufweist, welche die Struktur davon bilden, entweder in den Ribonukleosiden oder in den Internukleotidbindungen, wobei die chemische Modifikation eine Modifikation aus der Gruppe gebildet durch die Addition einer Phosphatgruppe am 5'-Ende der Oligoribonukleotidsequenz, den Austausch der 2'-Hydroxylgruppen in den Komponentenribosen der Oligoribonukleotidsequenz durch 2'-O-Methylgruppen, die Substitution von Ribonukleosidanaloga wie die N3'-P5'-Phosphoramidate für Ribonukleoside und den Austausch der Internukleotidphosphatbindungen durch andere Bindungen wie Phosphorthioat oder Methylphosphonat ist.

7.  Doppelstrang-Oligoribonukleotid gebildet durch das Verbinden der Oligoribonukleotidsequenzen nach Anspruch 1 durch Homologie, wobei der entstehende Doppelstrang an den 3'-Enden davon durch zwei ungepaarte Abschluss-nukleotide uu oder tt:

```
5'-gcgaauuccuagacaccuguu-3'    (SEQ. ID. NR. 7)
3'-uucgcuuaaggaucuguggac-5'


5'-gcgaauuccuagacaccugtt-3'    (SEQ. ID. NR. 8)
3'-ttcgcuuaaggaucuguggac-5'
```

erweitert ist.

8.  Doppelstrang-Oligoribonukleotid gebildet durch das Verbinden der erweiterten oligoribonukleotidsequenzen nach Anspruch 2 durch Homologie, wobei der entstehende Doppelstrang an den 3'-Enden davon durch zwei ungepaarte Abschlussnukleotide uu oder tt erweitert ist.

9.  Doppelstrang-Oligoribonukleotid gebildet durch das Verbinden von zwei modifizierten Oligoribonukleotidsequenzen nach Anspruch 3 durch Homologie, wobei der entstehende Doppelstrang an den 3'-Enden davon durch zwei ungepaarte Abschlussnukleotide uu oder tt erweitert ist.

10. Doppelstrang-Oligoribonukleotid gebildet durch das Verbinden von zwei verlängerten Oligoribonukleotidsequenzen nach Anspruch 4 durch Homologie, wobei der entstehende Doppelstrang an den 3'-Enden davon durch zwei ungepaarte Abschlussnukleotide uu oder tt erweitert ist.

11. Doppelstrang-Oligoribonukleotid nach einem der Ansprüche 7 bis 10, **dadurch gekennzeichnet, dass** die Oligoribonukleotidsequenz, die erweiterte Oligoribonukleotidsequenz, die modifizierte Oligoribonukleotidsequenz oder die verlängerte Oligoribonukleotidsequenz die Verbindung von mindestens einem Peptid, einem Zucker, einem Lipid oder einem Polymer aufweist.

12. Doppelstrang-Oligoribonukleotid nach einem der Ansprüche 7 bis 11, **dadurch gekennzeichnet, dass** die Oligoribonukleotidsequenz, die erweiterte Oligoribonukleotidsequenz, die modifizierte Oligoribonukleotidsequenz oder die verlängerte Oligoribonukleotidsequenz mindestens eine chemische Modifikation in einem oder einem anderen der Elemente aufweist, welche die Struktur davon bilden, entweder in den Ribonukleosiden oder in den Internukleotidbindungen, wobei die chemische Modifikation eine Modifikation aus der Gruppe gebildet durch die Addition einer Phosphatgruppe am 5'-Ende der Oligoribonukleotidsequenz, den Austausch der 2'-Hydroxylgruppen in den Komponentenribosen der Oligoribonukleotidsequenz durch 2'-O-Methylgruppen, die Substitution von Ribonukleosidanaloga wie die N3'-P5'-Phosphoramidate für Ribonukleoside und den Austausch der Internukleotidphosphatbindungen durch andere Bindungen wie Phosphorthioat oder Methylphosphonat ist.

13. Doppelstrang-Oligoribonukleotid nach einem der Ansprüche 7 bis 12, **dadurch gekennzeichnet, dass** die Oligoribonukleotidsequenzen, die erweiterten Oligoribonukleotidsequenzen, die modifizierten Oligoribonukleotidsequenzen oder die verlängerten Oligoribonukleotidsequenzen an einem der Enden davon durch eine Schleife oder einen Linker von 2 bis 25 Nukleotiden zum Bilden einer shRNA kovalent miteinander verbunden sind.

14. Pharmazeutische Zusammensetzung, welche eine Oligoribonukleotidsequenz nach einem der Ansprüche 1 bis 6 umfasst.

**15.** Pharmazeutische Zusammensetzung, welche ein Doppelstrang-Oligoribonukleotid nach einem der Ansprüche 7 bis 13 umfasst.

**16.** Gentransfervektor, welcher eine Oligoribonukleotidsequenz nach einem der Ansprüche 1 bis 6 exprimiert.

**17.** Gentransfervektor, welcher ein Doppelstrang-Oligoribonukleotid nach einem der Ansprüche 7 bis 13 exprimiert.

**18.** Verwendung einer Oligoribonukleotidsequenz nach einem der Ansprüche 1 bis 6 für das Herstellen eines Medikamentes.

**19.** Verwendung eines Doppelstrang-Oligoribonukleotids nach einem der Ansprüche 7 bis 13 für das Herstellen eines Medikamentes.

**20.** Verwendung einer Oligoribonukleotidsequenz nach einem der Ansprüche 1 bis 6 für das Herstellen eines Medikamentes zur Behandlung einer Erkrankung aus der Gruppe gebildet durch Abstoßungsphänomene bei Organ- oder Gewebetransplantaten, akute Entzündungsprozesse, chronische Entzündungsprozesse, Prozesse mit Kostimulation von Lymphozyten, Allergiemanifestationen, Autoimmunmanifestationen oder kardiovaskuläre Manifestationen, Infektionsprozesse, rheumatologische Manifestationen und Krebs.

**21.** Verwendung eines Doppelstrang-Oligoribonukleotids nach einem der Ansprüche 7 bis 13 für das Herstellen eines Medikamentes zur Behandlung einer Erkrankung aus der Gruppe gebildet durch Abstoßungsphänomene bei Organ- oder Gewebetransplantaten, akute Entzündungsprozesse, chronische Entzündungsprozesse, Prozesse mit Kostimulation von Lymphozyten, Allergiemanifestationen, Autoimmunmanifestationen oder kardiovaskuläre Manifestationen, Infektionsprozesse, rheumatologische Manifestationen und Krebs.

**22.** Verwendung einer Oligoribonukleotidsequenz nach einem der Ansprüche 1 bis 6 für das Herstellen eines Medikamentes zur Behandlung einer Erkrankung aus der Gruppe gebildet durch allergische Dermatitis, Psoriasis, Arteriosklerose, Multiple Sklerose, Lungenfibrose, Vireninfektionen, Bakterieninfektionen, Asthma, Diabetes Typ I, Lyme-Krankheit, Crohn-Krankheit, ulzeröse Kolitis, Lupus, Thyreoiditis, Arthritis, Leukämien und Lymphome.

**23.** Verwendung eines Doppelstrang-Oligoribonukleotids nach einem der Ansprüche 7 bis 13 für das Herstellen eines Medikamentes zur Behandlung einer Erkrankung aus der Gruppe gebildet durch allergische Dermatitis, Psoriasis, Arteriosklerose, Multiple Sklerose, Lungenfibrose, Vireninfektionen, Bakterieninfektionen, Asthma, Diabetes Typ I, Lyme-Krankheit, Crohn-Krankheit, ulzeröse Kolitis, Lupus, Thyreoiditis, Arthritis, Leukämien und Lymphome.

**24.** Verwendung einer Oligoribonukleotidsequenz nach einem der Ansprüche 1 bis 6 für das Herstellen eines Medikamentes zur Behandlung neurodegenerativer Störungen.

**25.** Verwendung eines Doppelstrang-Oligoribonukleotids nach einem der Ansprüche 7 bis 13 für das Herstellen eines Medikamentes zur Behandlung neurodegenerativer Störungen.

**Revendications**

**1.** Séquence oligoribonucléotidique du groupe formé par les séquences oligoribonucléotidiques suivantes:
5'-gcgaauuccuagacaccug-3' (SEQ ID N° 1)
et
5'-caggugucuaggaauucgc-3' (SEQ ID N° 2)
ces séquences oligoribonucléotidiques étant homologues de la région comprise entre les nucléotides 241 et 259 dans l'ADNc codant le récepteur de CD40 humain, conforme au numéro d'accès GeneBank X60592.

**2.** Séquence oligoribonucléotidique selon la revendication 1, **caractérisée en ce qu'**elle est étendue à l'une de ses extrémités d'au plus quatre ribonucléotides additionnels au total, ce qui forme une séquence oligoribonucléotidique étendue, dans laquelle lesdits ribonucléotides additionnels sont homologues de la région correspondante comprise entre les nucléotides 237 et 263 dans l'ADNc codant le récepteur de CD40 humain, conforme au numéro d'accès GeneBank X60592.

**3.** Séquence oligoribonucléotidique comprenant une sous-séquence oligoribonucléotidique du groupe formé par les sous-séquences oligoribonucléotidiques suivantes :
5'-uccuagacaccug-3' (SEQ ID N° 3)
5'-gcgaauuccuaga-3' (SEQ ID N° 4)
5'-ucuaggaauucgc-3' (SEQ ID N° 5)
5'-caggugucuagga-3' (SEQ ID N° 6)
et qui de plus comprend au maximum 12 ribonucléotides additionnels ajoutés à l'une de ses extrémités ou aux deux extrémités simultanément, ce qui forme une séquence oligoribonucléotidique modifiée, de façon que ladite séquence oligoribonucléotidique modifiée soit homologue de la région_comprise entre les nucléotides 230 et 270 dans l'ADNc codant le récepteur de CD40 humain, conforme au numéro d'accès GeneBank X60592.

4. Séquence oligoribonucléotidique selon l'une quelconque des revendications 1 à 3, **caractérisée en ce qu'**elle comprend de plus un à trois ribonucléotides terminaux à son extrémité 3', ce qui forme une séquence nucléotidique allongée, dans laquelle lesdits ribonucléotides terminaux ne sont pas homologues de la région correspondante entre les nucléotides 230 et 270 dans l'ADNc codant le récepteur de CD40 humain, conforme au numéro d'accès GeneBank X60592.

5. Séquence oligoribonucléotidique selon l'une quelconque des revendications 1 à 4, **caractérisée en ce que** ladite séquence oligoribonucléotidique, ladite séquence oligoribonucléotidique étendue, ladite séquence oligoribonucléotidique modifiée, ou ladite séquence oligoribonucléotidique allongée a la jonction d'au moins un peptide, un sucre, un lipide ou un polymère.

6. Séquence oligoribonucléotidique selon l'une quelconque des revendications 1 à 5, **caractérisée en ce que** ladite séquence oligoribonucléotidique, ladite séquence oligoribonucléotidique étendue, ladite séquence oligoribonucléotidique modifiée, ou ladite séquence oligoribonucléotidique allongée a une modification chimique dans l'un ou l'autre des éléments formant la structure de celle-ci, soit dans les ribonucléotides soit dans les liaisons internucléotidiques, dans laquelle ladite modification chimique est une modification du groupe formé par l'addition d'un groupe phosphate à l'extrémité 5' de la séquence oligoribonucléotidique, le remplacement par des groupes 2'-0-méthyle des groupes 2'-hydroxyle dans les riboses constitutifs de la séquence oligoribonucléotidique, la substitution de ribonucléotides par des analogues de ribonucléoside tels que les phosphorimidates N3'→P5', et le remplacement des liaisons phosphates internucléotidiques par d'autres liaisons telles que phosphorothioate ou méthylphosphonate.

7. Oligoribonucléotide duplex formé par la jonction par homologie des séquences oligoribonucléotidiques selon la revendication 1, dans lequel le double brin résultant est étendu aux extrémités 3' de celui-ci par deux nucléotides terminaux non appariés uu ou tt :

```
5'-gcgaauuccuagacaccuguu-3'   (SEQ ID N° 7)
3'-uucgcuuaaggaucuguggac-5'
```

```
5'-gcgaauuccuagacaccugtt-3'   (SEQ ID N° 8)
3'-ttcgcuuaacgaucuguggac-5'
```

8. Oligoribonucléotide duplex formé par la jonction par homologie des séquences oligoribonucléotidiques étendues selon la revendication 2, dans lequel le double brin résultant est étendu aux extrémités 3' de celui-ci par deux nucléotides terminaux non appariés uu ou tt.

9. Oligoribonucléotide duplex formé par la jonction par homologie des séquences oligoribonucléotidiques modifiées selon la revendication 3, dans lequel le double brin résultant est étendu aux extrémités 3' de celui-ci par deux nucléotides terminaux non appariés uu ou tt.

10. Oligoribonucléotide duplex formé par la jonction par homologie des séquences oligoribonucléotidiques allongées selon la revendication 4, dans lequel le double brin résultant est étendu aux extrémités 3' de celui-ci par deux nucléotides terminaux non appariés uu ou tt.

11. Oligoribonucléotide duplex selon l'une quelconque des revendications 7 à 10, **caractérisé en ce que** ladite séquence oligoribonucléotidique, ladite séquence oligoribonucléotidique étendue, ladite séquence oligoribonucléotidique modifiée, ou ladite séquence oligoribonucléotidique allongée a la jonction d'au moins un peptide, un sucre, un lipide ou un polymère.

12. Oligoribonucléotide duplex selon l'une quelconque des revendications 7 à 11, **caractérisé en ce que** ladite séquence oligoribonucléotidique, ladite séquence oligoribonucléotidique étendue, ladite séquence oligoribonucléotidique modifiée, ou ladite séquence oligoribonucléotidique allongée a au moins une modification chimique dans l'un ou l'autre des éléments formant la structure de celle-ci, soit dans les ribonucléosides soit dans les liaisons internucléotidiques, dans laquelle ladite modification chimique est une modification du groupe formé par l'addition d'un groupe phosphate à l'extrémité 5' de la séquence oligoribonucléotidique, le remplacement par des groupes 2'-O-méthyle des groupes 2'-hydroxyle dans les riboses constitutifs de la séquence oligoribonucléotidique, la substitution de ribonucléotides par des analogues de ribonucléoside tels que les phosphorimidates N3'→P5', et le remplacement des liaisons phosphates internucléotidiques par d'autres liaisons telles que phosphorothioate ou méthylphosphonate.

13. Oligoribonucléotide duplex selon l'une quelconque des revendications 7 à 12, **caractérisé en ce que** lesdites séquences oligoribonucléotidiques, lesdites séquences oligoribonucléotidiques étendues, lesdites séquences oligoribonucléotidiques modifiées, ou lesdites séquences oligoribonucléotidiques allongées sont jointes ensemble de façon covalente à l'une de leurs extrémités par l'intermédiaire d'une boucle ou d'un lieur de 2 à 25 nucléotides pour former un ARNsh.

14. Composition pharmaceutique comprenant une séquence oligoribonucléotidique selon l'une quelconque des revendications 1 à 6.

15. Composition pharmaceutique comprenant un oligoribonucléotide duplex selon l'une quelconque des revendications 7 à 13.

16. Vecteur de transfert de gène exprimant une séquence oligoribonucléotidique selon l'une quelconque des revendications 1 à 6.

17. Vecteur de transfert de gène exprimant un oligoribonucléotide duplex selon l'une quelconque des revendications 1 à 13.

18. Utilisation d'une séquence oligoribonucléotidique selon l'une quelconque des revendications 1 à 6 pour la préparation d'un médicament.

19. Utilisation d'un oligonucléotide duplex selon l'une quelconque des revendications 7 à 13 pour la préparation d'un médicament.

20. Utilisation d'une séquence oligoribonucléotidique selon l'une quelconque des revendications 1 à 6, pour la préparation d'un médicament destiné au traitement d'une maladie du groupe formé par le phénomène de rejet de greffes d'organe ou de tissu, les processus inflammatoires aigus, les processus inflammatoires chroniques, les processus avec co-stimulation de lymphocytes, les manifestations allergiques, les manifestations auto-immunes ou les manifestations cardiovasculaires, les processus infectieux, les manifestations rhumatologiques et le cancer.

21. Utilisation d'un oligoribonucléotide duplex selon l'une quelconque des revendications 7 à 13, pour la préparation d'un médicament destiné au traitement d'une maladie du groupe formé par le phénomène de rejet de greffes d'organe ou de tissu, les processus inflammatoires aigus, les processus inflammatoires chroniques, les processus avec co-stimulation de lymphocytes, les manifestations allergiques, les manifestations auto-immunes ou les manifestations cardiovasculaires, les processus infectieux, les manifestations rhumatologiques et le cancer.

22. Utilisation d'une séquence oligoribonucléotidique selon l'une quelconque des revendications 1 à 6, pour la préparation d'un médicament destiné au traitement d'une maladie du groupe formé par la dermatite allergique, le psoriasis, l'artériosclérose, la sclérose en plaques, la fibrose pulmonaire, les infections virales, les infections bactériennes, l'asthme, le diabète de type I, la maladie de Lyme, la maladie de Crohn, la recto-colite hémorragique, le lupus, la thyroïdite, l'arthrite, les leucémies et les lymphomes.

23. Utilisation d'un oligoribonucléotide duplex selon l'une quelconque des revendications 7 à 13, pour la préparation d'un médicament destiné au traitement d'une maladie du groupe formé par la dermatite allergique, le psoriasis, l'artériosclérose, la sclérose en plaques, la fibrose pulmonaire, les infections virales, les infections bactériennes, l'asthme, le diabète de type I, la maladie de Lyme, la maladie de Crohn, la recto-colite hémorragique, le lupus, la thyroïdite, l'arthrite, les leucémies et les lymphomes.

24. Utilisation d'une séquence oligoribonucléotidique selon l'une quelconque des revendications 1 à 6, pour la préparation d'un médicament destiné au traitement de troubles neurodégénératifs.

25. Utilisation d'un oligoribonucléotide duplex selon l'une quelconque des revendications 7 à 13, pour la préparation d'un médicament destiné au traitement de troubles neurodégénératifs.

FIG. 1

FIG. 2

EP 1 614 751 B1

FIG. 3

FIG. 4

FIG. 7

FIG. 5

FIG. 8

FIG. 6

FIG. 9

FIG. 10

FIG. 11

FIG. 12

EP 1 614 751 B1

FIG. 13

FIG. 14

FIG. 15

FIG. 16

FIG. 17

FIG. 18

FIG. 19